# FASCICULE DE BREVET EUROPEEN

(11) **EP 0 596 881 B1**
(45) Date de publication et mention de la délivrance du brevet: **19.03.1997**
(21) Numéro de dépôt: 91914319.8
(22) Date de dépôt: 01.08.1991
(51) Int. Cl.: C12N 15/86, C12N 5/10, C12N 15/24, C12N 15/27, C12N 15/16

(54) **EXPRESSION DANS DES LIGNEES LYMPHOBLASTOIDES HUMAINES NON-TUMORALES AVEC UN VECTEUR INTEGRATIF**
Expression in nicht-tumorbildenden menschlichen Lymphoblasten Zellinien mit einem integrierenden Vektor
EXPRESSION IN NON-TUMORAL HUMAN LYMPHOBLASTOID LINES WITH AN INTEGRATIVE VECTOR

(43) Date de publication de la demande: 18.05.1994
(73) Titulaire: FONDATION NATIONALE DE TRANSFUSION SANGUINE, 75739 Paris Cédex 15 (FR)
(72) Inventeur: BALLAY, Annick, F-78150 Le Chesnay (FR); BOFFA, Georges, F-91120 Palaiseau (FR); CARTRON, Jean-Pierre, F-75007 Paris (FR); CHRETIEN, Stany, F-75015 Paris (FR); LAMBIN, Patrick, F-75015 Paris (FR); LOPEZ, Claude, F-94360 Bry-sur-Marne (FR); PRIGENT, Sylvie, F-91440 Bures-sur-Yvette (FR); SALMON, Charles, F-75012 Paris (FR)
(74) Mandataire: Lepeudry-Gautherat, Thérèse (FR)
(86) Numéro de dépôt international: FR9100636
(87) Numéro de publication internationale: WO9303163

(56) Documents cités:
- WO-A-88/04691
- PROCEEDINGS OF THE NATL. ACADEMY OF SCIENCES USA, vol. 83, no. 13, 1986, Washington, DC (US); C. SVENSSON et al., pp. 4690-4694
- CHEMICAL ABSTRACTS, vol. 103, 1985, Columbus, OH (US); C. SVENSSON et al., p. 205, no. 136210; and G. LUTFALLA et al., p. 117, no. 48859j
- BIOTECHNOLOGY, vol. 8, no. 6, juin 1990, New York, NY (US); M. OKAMOTO et al., pp. 550-553
- PROCEEDINGS OF THE NATL. ACADEMY OF SCIENCES USA, vol. 78, no. 4, avril 1981, Washington, DC (US); R.C. MULLIGAN et al., pp. 2072-2076
- GENE, vol. 76, 1989, Amsterdam (NL); H. YANAGI et al., pp. 19-26
- GENE, vol. 101, no. 2, 30 mai 1991, Amsterdam (NL); M. LEVRERO et al., pp. 195-202
- Chemical Abstracts, vol. 103, 1985, Columbus, Ohio, US; abstract No. 48859J, page 117; column 1; vol. 11, No. 3, 1985, pages 223-238; LUTFALLA, G. et al.

## Description

L'invention concerne l'utilisation de lignées cellulaires humaines pour la production, à l'échelle industrielle, de protéines hétérologues, plus particulièrement de protéines d'intérêt thérapeutique, après manipulation génétique de leur séquence codante.

Plus particulièrement, l'invention concerne l'utilisation de cellules lymphoblastoïdes humaines, immortalisées in vitro par le virus d'Epstein-Barr et sélectionnées pour leur absence de caractère tumoral ou tumorigène. Pour assurer l'expression de protéines hétérologues par lesdites cellules, la séquence codante de ces protéines est insérée dans un plasmide vecteur génétiquement manipulé, de type intégratif et portant une cassette d'expression conçue pour permettre l'expression desdites protéines dans les cellules lymphoblastoïdes transformées, clonées et sélectionnées, et ayant intégré la cassette d'expression dans l'ADN chromosomique cellulaire.

L'invention concerne aussi la conception et la construction de ce plasmide vecteur destiné à assurer une production optimale des protéines hétérologues par les cellules lymphoblastoïdes transformées.

La production de protéines d'intérêt thérapeutique difficiles à purifier à partir de sources naturelles où elles sont présentes en petites quantités ou dont les sources sont potentiellement dangereuses comme les tumeurs ou le plasma sanguin éventuellement contaminé par des virus, justifie encore de nombreux efforts de recherche et développement en cultures de cellules in vitro, dans des conditions de culture bien standardisées et offrant des garanties d'innocuité.

Cependant l'expression de molécules de grande taille et dont l'activité biologique dépend de la structure spatiale et de la maturation correcte (glycosylation, sialylation, etc..) n'est pas possible dans n'importe quel type de cellule, en particulier dans les microorganismes, dont la fermentation industrielle est simple. Par contre dans les cellules eucaryotes, si la maturation des molécules pose moins de problèmes, c'est la culture même des cellules qui pose des problèmes à l'échelle industrielle, la plupart des cellules non tumorales ne se multipliant que sous forme de monocouche, adhérant au récipient de culture. Des progrès importants ont été apportés par la mise au point de techniques de culture sur microporteurs et en bioréacteurs mais elles ne conviennent pas à tous les types de cellules et posent des problèmes si les protéines ne sont pas excrétées dans le milieu de culture. D'autre part des cellules comme les Vero qui sont particulièrement bien adaptées à ce mode de culture sont très réfractaires aux manipulations génétiques.

La recherche de nouvelles lignées cellulaires et de vecteurs adaptés pour permettre la manipulation génétique de celles-ci justifie donc toujours d'importants efforts de recherche.

C'est pourquoi la Demanderesse a envisagé l'utilisation de lignées lymphoblastoïdes humaines semblables à celles qu'on utilise déjà pour la production d'anticorps monoclonaux mais cette utilisation nécessite la mise au point de vecteurs de clonage et d'expression particulièrement adaptés à ces cellules.

Une lignée lypmphoblastoïde humaine, la lignée Namalwa, a déjà été utilisée pour produire une protéine hétérologue ; elle se multiplie bien en suspension et sécrète la protéine hétérologue dans le milieu où on peut la récupérer, sous forme biologiquement active (Yanagi et al. Gene 1989,76,19 et DNA 1989,8,419) mais cette lignée est dérivée d'un lymphome de Burkitt et présente un haut degré de tumorigénicité.

C'est pourquoi l'objectif de la présente invention est l'utilisation de cellules lymphoblastoïdes humaines non tumorales et non tumorigènes.

De telles cellules sont connues : par transformation avec le virus d'Epstein-Barr (EBV) on peut immortaliser des lymphocytes B du sang d'un donneur sain quelconque, et sélectionner des lignées qui ne synthétisent que l'antigène nucléaire EBNA-1 de l'EBV (Thoda et al. Cancer Res. 38, 1978, 3560). De telles lignées prolifèrent de manière indéfinie en culture mais ne sont pas tumorales, ce qu'on vérifie par leur absence de capacité de former des colonies en agar, et elles ne sont pas tumorigènes sur la souris "nu-nu" (Gurtsevitch et al, Int J. Cancer 47, 87, 1988, Tursz, Medecine/Sciences 6, Suppl.42, 1989).

La Demanderesse utilise déjà des lignées de ce type pour produire des anticorps monoclonaux. Un milieu de culture pauvre en protéines a été mis au point pour faciliter la purification des molécules sécrétées (brevet F 2 600 076).

Ce type de cellule a déjà servi de modèle pour exprimer des protéines hétérologues dont le gène est inséré dans un réplicon autonome qui se maintient à l'état épisomique dans la cellule parce qu'il porte l'origine de réplication de l'EBV qui se retrouve sous le contrôle de la protéine EBNA-1 de l'EBV qui a servi à immortaliser la lignée cellulaire (Kioussis et al. EMBO J.6, 1987, 355 ; Young et al. Gene 62, 1988, 171 ; Jalanko et al. Gene 78, 1989, 287). Toutefois, l'état épisomique du réplicon ne permet pas de garantir la stabilité de son nombre de copies par cellule et donc la capacité de production de la protéine hétérologue au cours du temps, surtout à une échelle industrielle qui implique un très grand nombre de cycles de multiplication cellulaire.

C'est pourquoi la présente invention concerne également la mise au point, par manipulation génétique, d'un vecteur comportant tous les éléments nécessaires à l'expression d'une protéine hétérologue dans les cellules lymphoblastoïdes humaines mais également des éléments d'ADN favorisant l'intégration de cet ensemble dans l'ADN chromosomique de la cellule réceptrice.

Ainsi la présente invention concerne l'utilisation de lignées de cellules lymphoblastoïdes humaines, immortalisées in vitro par l'EBV et sélectionnées pour leur absence de caractère tumoral ou tumorigène, lesdites cellules pouvant être dérivées des lymphocytes B d'un donneur sain quelconque ou, parmi de telles lignées, on peut choisir une lignée particulière et bien caractérisée, déposée à l'ATCC sous la référence CCL 156 RPMI 1788.

L'utilisation selon l'invention permet la production, à l'échelle industrielle, de protéines hétérologues codées par un segment d'ADN adapté pour être inséré dans un plasmide vecteur génétiquement manipulé, de type intégratif et portant une cassette d'expression comprenant tous les éléments permettant l'expression desdites protéines hétérologues après intégration de ladite cassette d'expression dans l'ADN chromosomique des cellules lymphoblastoïdes.

La présente invention concerne également la conception et la construction du plasmide vecteur destiné à l'utilisation des cellules lymphoblastoïdes pour la production de protéines hétérologues. Ledit plasmide comprend, outre les éléments d'ADN bactérien nécessaires à son amplification dans un hôte de type E.Coli et qui dérivent du plasmide de départ pMLP10 (Ballav et al. Hepadna Viruses, 1987, 481 Ed. Alan R. Liss),
- les éléments qui constituent une cassette d'expression permettant l'insertion d'un ADN hétérologue quelconque en vue de son expression dans les cellules lymphoblastoïdes humaines,
- la séquence d'ADN de l'Adenovirus 5 codant pour les ARN VA I et VA II pour optimaliser la traduction des ARN messagers (Mathews, Cell 6, 223, 1975 ; Soderland et al, Cell 1, 585, 1976 ; Schneider et al, Cell 37, 291, 1984, Svensson et al. EMBO J. 4, 957, 1985) ;
- le plasmide comprend en outre un gène de sélection manipulé pour être efficace dans les cellules eucaryotes : on peut choisir l'ADN codant pour la xanthine-guanine phosphoribosyl transférase (XGPRT) de E.coli placé sous le contrôle du promoteur et des signaux de maturation du gène TK du virus Herpes simplex 1 (HSV1) (Mulligan Berg. Proc. Natl. Acad. Sci. USA 98, 2072, 1981) ; on peut également choisir l'ADNc de la dihydrofolate réductase de souris (DHFR) qui permet une amplification génique en présence de méthotrexate (Alt et al, J. Biol. Chem.253, 1357, 1978) on peut aussi envisager l'utilisation simultanée des deux gènes de sélection, insérés à des points distincts du plasmide vecteur.
- le plasmide vecteur comprend également des éléments d'ADN sélectionnés pour favoriser l'intégration de l'ADN plasmidique dans l'ADN chromosomique de la cellule lymphoblastoïde. Ces éléments d'ADN seront plus particulièrement choisis parmi les fragments d'ADN mitochondrial de souris qui ont été décrits comme favorisant la multimérisation en tandem d'ADN hétérologue au cours de l'intégration dans le génome de cellules de mammifères (Lutfalla et al. Somatic cell and Mol. Genetics, 11, 223, 1985).

La cassette d'expression comprend un ensemble d'éléments constituant une unité de transcription fonctionnelle dans les cellules choisies et plus particulièrement :
- en amont de la position qui recevra la séquence d'ADN hétérologue, des éléments de régulation d'Adenovirus humains comprenant une séquence activatrice, un promoteur et une séquence "leader",
- et, en aval de cette position, un signal de polyadénylation dérivé du virus SV 40.

Les éléments dérivés d'Adenovirus humains sont de préférence :
- le promoteur de l'Adenovirus 5 (Lavery et al. J. Virol. 61, 14666, 1987) placé sous le contrôle d'une séquence activatrice de la transcription, située en amont du promoteur, et de préférence la séquence activatrice E1A de l'Adenovirus 5,
- le promoteur majeur tardif (MLP) de l'Adenovirus 2 couplé à une copie ADNc de la séquence "leader" tripartite de l'Adenovirus 2, ce qui permet une augmentation de l'efficacité de la traduction des ARN messagers. Ces divers éléments indépendants sont décrits par Berkner (Biotechniques 6, 616, 1988).
Ce choix n'est pas limitatif : d'autres éléments dérivés d'Adenovirus peuvent aussi être utilisés comme le promoteur majeur tardif de l'Adenovirus 5 et les "enhancers" du BKV (Grinnell et al, Mol Cell. Biol. 6, 3596-3605, 1986) ou du SV40 (Zenke et al, EMBO J. 5, 387-397, 1986), et en particulier de leur association en tandem (Berg et al., Nucl. Acid. Res, 16, 1635, 1988).

La zone centrale de la cassette d'expression, destinée à l'insertion de la séquence d'ADN hétérologue a été conçue pour offrir des sites de restriction rares pour favoriser cette manipulation d'insertion, et plus particulièrement, les sites Not I, Bst E II, Bgl II, Mlu I.

L'invention est plus particulièrement destinée à l'utilisation des cellules décrites, avec le plasmide vecteur décrit, pour produire des protéines d'intérêt thérapeutique difficiles à purifier à partir de sources naturelles. L'invention comprend donc l'insertion des ADN codant pour ces protéines hétérologues dans la cassette d'expression telle que décrite plus haut. Le plasmide vecteur est ensuite introduit dans les cellules par électroporation (Potter et al. Proc. Natl. Acad. Sci. USA 81, 7161, 1984).

Comme premier ADN utilisé à titre de modèle pour prouver la faisabilité technique des productions proposées, on a utilisé l'ADN de l'érythropoïétine humaine (qui sera notée Epo ou Epo HR pour erythropoïétine humaine recombinante). On peut aussi utiliser un ADN d'une protéine analogue présentant l'activité de l'érythropoïétine.

La protéine naturelle est produite dans les reins et circule en très faible quantité dans le plasma. Elle joue un rôle essentiel dans la maturation des érythrocytes.

Elle comprend 166 acides aminés et de 40 à 50 % de glucides ce qui impose sa synthèse dans des cellules appropriées. Son extraction à partir d'urine de patients aplasiques (Mikake et al. J. Biol. Chem. 252 5558, 1977) et à partir de tumeurs rénales (Sherwood et al. Endocrinology 99, 504, 1976) sont décrites mais ne permettent pas une production industrielle.

La production d'Epo humaine recombinante a également été décrite dans divers types de cellules (COS, CHO, BHK, NIH ₃T₃) mais les inconvénients de ces différentes cellules ont déjà été évoqués plus haut.

Un deuxième modèle a été mis au point avec la séquence codante de l'interleukine 3 (IL-3).

L'IL-3 est une glycoprotéine de 20-26 kDa stimulant la prolifération et la différenciation des progéniteurs hématopoiétiques appartenant aux lignées érythroïdes, mégacaryocytaires, granulo-macrophagiques, éosinophiles et basophiles, dont l'ADNc humain a été cloné par Yang et al (Cell 47,3-10,1986). Chez la souris, l'IL-3 est impliquée dans l'autorenouvellement et la différenciation de la cellule souche multipotente (CFU-S), en synergie avec l'IL-1. L'IL-3 possède également, en synergie avec les cytokines spécifiques de chaque lignée, une activité sur la croissance et les fonctions de cellules différenciées (macrophages et mastocytes, polynucléaires neutrophiles et éosinophiles). A cause de son rôle dans la stimulation des cellules souches hématopoiétiques, l'IL-3 pourrait trouver une application thérapeutique dans le traitement des aplasies médullaires (Shrader, Annu. Rev. Immunol. 4, 205-230, 1986 ; Mizel, FASEB J. 3, 2379, 1989 ; Tigaud et al., medecine/sciences 7, 444, 1991). Il a été montré aussi que l'IL-3 stimule l'hématopoièse chez les primates, en synergie avec le GM-CSF (Donahue et al., Science 241, 1820, 1988).

Un troisième modèle a été mis au point avec la séquence codante du GM-CSF (granulocyte-macrophage colony stimulating factor). Le GM-CSF est aussi une multipoiétine, c'est-à-dire un facteur de croissance capable, comme l'IL-3, de stimuler la croissance et la différenciation de plusieurs lignées hématopoiétiques (granulo-monocytaires, érythroides et colonies mixtes) et d'agir sur les fonctions des cellules différenciées (macrophages). Le gène humain du GM-CSF a été cloné par plusieurs équipes (Wong et al, Science 228, 810, 1985 ; Lee et al, Proc. Natl. Acad. Sci. USA 82, 4360, 1985 ; Cantrell et al, Proc. Natl Acad; Sci USA 82, 6250, 1985 ; Miyatake et al, EMBO J. 4, 2561, 1985) et cette molécule fait l'objet d'études de développement clinique chez l'homme dans le traitement des aplasies médullaires, des leucopénies d'origine virale (EBV, HIV) et le traitement des cytopénies induites par les chimiothérapies anti-néoplasiques (Clark et Kamen, Science 236, 1229, 1987 ; Groopman et al, N. Engl. J. Med. 321, 1449, 1989 ; Applebaum et al, Sem. Hematol. 26, 7, 1989 ; Solal-Céligny, médecine/Sciences 4, 231, 1991 ; Tigaud et al., médecine/sciences 7, 444, 1991).

La présente invention couvre également les lignées cellulaires après leur transformation stable par un plasmide vecteur tel que décrit et qui ont été clonées et sélectionnées par criblage, parmi de nombreux clones transformés, pour leur taux élevé de sécrétion de la protéine hétérologue choisie. Les procédés de récolte et de purification sont adaptés à chacune des protéines.

Les exemples suivants sont destinés à illustrer l'invention sans toutefois en limiter la portée.

Les 2 figures suivantes permettent de mieux comprendre les constructions génétiques :

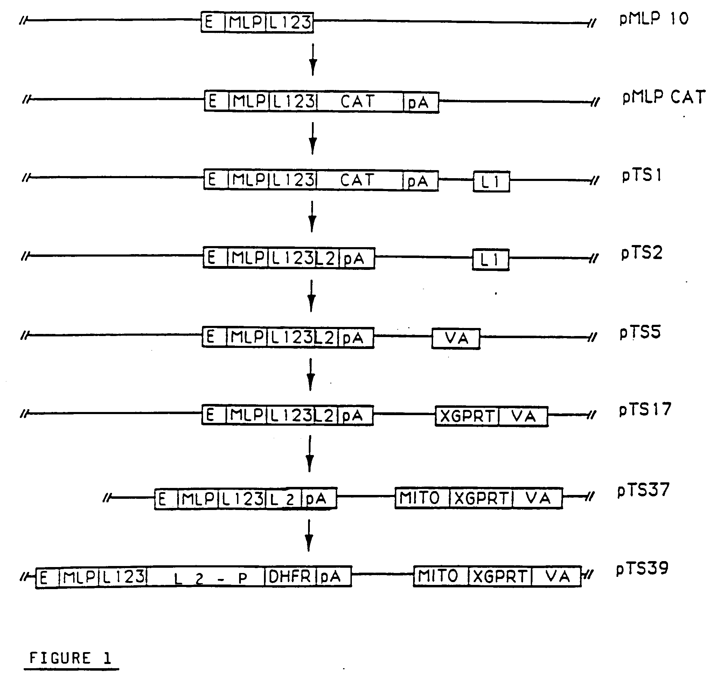

Figure 1 : Schéma de la construction du vecteur pTS39.
   Toutes les constructions dérivent du plasmide pMLP10. Les abréviations suivantes sont utilisées pour désigner les différents fragments d'ADN : E : séquence activatrice du gène E1A d'Ad5 ; MLP : promoteur majeur tardif d'Ad2 ; L : copie ADNc de la séquence leader tripartite d'Ad2 ; CAT : gène bactérien codant pour la chloramphénicol acétyl transférase ; L1 : polylinker du pUC18 ; L2 : oligonucléotide de synthèse ; P : région codante de la protéine hétérologue ; pA : signal de polyadénylation du virus SV40 ; XGPRT : gène bactérien codant pour la xanthine guanine phosphoryosyl transférase ; VA : gènes d'Ad5 codant pour les ARN VA ; mito : fragment d'ADN murin d'origine mitochondriale ; DHFR : ADNc murin codant pour la dihydrofolate reductase.

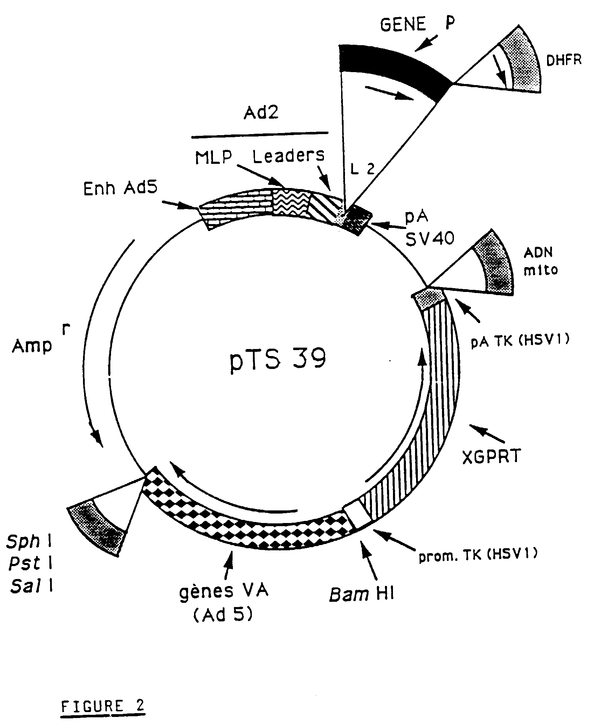

Figure 2 : Schéma du vecteur d'expression de type intégratif pTS39.
   ADN mito : fragment d'ADN murin d'origine mitochondriale. Un second gène de sélection (DHFR) est placé en position bicistronique en aval de la région codante P.
   Autres abréviations : comme figure 1.

### Exemple 1 : construction du vecteur intégratif

Le vecteur intégratif pTS39 représenté sur la figure 2 a été construit par les étapes successives suivantes, schématisées dans la figure 1.
- Le plasmide de départ est le pMLP10 (Ballay et al. Hepadna Viruses, 481, 1987, Ed. Alan R. Liss).
- Le plasmide pMLPCAT dérive du pMLP10 par insertion du fragment HindIII-BamHI du pHp34-CAT contenant le gène CAT et les signaux de maturation de SV40 (P. Gilardi et al, Nucleic Acid Research 20. 7877-7887, 1984) entre les sites HindIII et BamHI du pMLP10.
- Le plasmide pTS1 a ensuite été obtenu par insertion du polynker EcoRI-HindIII de pUC18 au niveau du site NruI du pMLPCAT.
- Le plasmide pTS2 dérive du pTS1 par insertion de l'oligonucléotide de synthèse suivant (noté L₂ dans les schémas) : 
-AGCTTGCGGCCGCGGTTACCAGATCTACGCGTGTT-3'
    -ACGCCGGCGCCAATGGTCTAGATGCGCACAA-5'
 contenant les sites rares NotI, SacII, BstEII, BglII, MluI entre les sites HindIII-HpaI du pTS1, et où sera insérée la séquence codante de la protéine hétérologue choisie (notée P).
- Le plasmide pTS5 est ensuite obtenu en insérant le fragment HindIII du pAG60VA (contenant les gènes VAI et VAII d'Ad5) au niveau du site XbaI du pTS2.
- Le plasmide pTS17 dérive du pTS5 par insertion du fragment PvuII de pAGT40 (contenant le gène XGPRT sous le contrôle du promoteur et des signaux de maturation du gène Tk du virus HSV1) au niveau du site SmaI de pTS5.
- Le plasmide pTS37 dérive de pTS17 par insertion du fragment EcoRI-PvuI du pδ1 contenant séquence d'ADN mitochondrial de souris au niveau du site SacI du pTS17.
- Enfin, le plasmide pTS39 est obtenu en insérant le fragment BamH1-BglII du pTG1086 (contenant la région codante du cDNA DHFR murin, qui constitue un moyen de pression sélective alternatif au XGPRT) au niveau du site BglII de pTS37, pour être placé en position bicistronique par rapport à la séquence codante P insérée dans l'oligonucléotide L₂.
- Le plasmide pTS39 sera noté pTS39-P quand la séquence codante d'une protéine hétérologue sera insérée dans l'oligonucléotide L₂ (et donc noté L₂-P sur le schéma).

### Exemple 2 : Utilisation du plasmide pTS39 pour produire l'érythropoïétine humaine dans les cellules lymphoblastoïdes

### 2.1. Isolement du gène de l'Epo

Une banque d'ADN génomique a été criblée avec un oligonucléotide 30-mer:5'-CGTGATATTCTCGGCCTCCTTGGCCTCCAA-3' déduit de la séquence du gène (Lin et al, Proc. Natl. Acad. Sci. USA 82, 7580, 1985). Cette sonde est complémentaire inversée de l'ARN messager et elle reconnaît la séquence de l'Epo en position 129-159 (la base No 1 représentant l'initiation de la traduction).

Le vecteur de clonage est EMBL 3, il contient des fragments insérés d'ADN humain de 12 à 20 kb. On étale 10⁷ clones répartis sur 10 boîtes de 15 cm de diamètre. Trois séries de filtres, répliques de ces boîtes, ont été lavées et hybridées avec les sondes oligonucléotidiques marquées à l'aide de la T4 polynucléotide kinase, et présentant une activité spécifique de 10⁹ cpm/µg, dans les conditions suivantes :
- Pré-hybridation : 3 SSC, 2 X Denhardt's, 0,005 % Tétrasodium disphosphate (Nappi), 100 µg/ml sperme de saumon, 52°C pendant 4 h.
- Hybridation : 6 SSC, 2 X Denhardt's, 0,05 % Nappi, 20 µg/ml tARN, 55°C pendant la nuit avec la sonde Epo (10⁶ cpm/ml par filtre).
- Lavage : 2XSSC, température laboratoire, 2 X SSC, 0,005 % Nappi, 3 x 20 min. à 52°C.
- Lavage supplémentaire en 1 X SSC, 0,05 % Nappi à 52°C pendant 20 min et/ou 0,1 SSC, 0,05 % Nappi à 52°C pendant 20min.

Quatre criblages successifs ont été réalisés pour aboutir à la caractérisation de 3 clones purs hybridant très fortement avec la sonde.

L'ADN de ces 3 phages a été amplifié, puis analysé. Afin de contrôler la présence du gène Epo dans son intégralité, la carte de restriction de ces 3 clones a été construite et la présence des extrémités 3' et 5' du gène Epo a été recherchée en utilisant deux sondes oligonucléotidiques reconnaissant les bornes de ce gène. Deux clones parmi les trois contenaient le gène Epo dans son intégralité. Des fragments de restriction contenant le gène Epo dans son intégralité ont été sous clonés dans un vecteur d'amplification du type pUC. Ainsi les deux fragments de restriction BamHI-HindIII de 5,4 kb et BglII-BglII de 4 kb ont été sous clonés.

### 2.2. Isolement de l'ADNc humain de l'Epo

Les ARN totaux d'une tumeur rénale humaine exprimant de façon constitutive l'Epo ont été isolés. A partir de ces ARN totaux la fraction ARN poly A+ a été isolée par passage sur colonne d'oligo-dT et clonée par la méthode PCR.

Deux sondes encadrant l'ADNc de l'Epo ont été choisies : . INTS 10 : 5'-CTGGAGTGTCCATGGGACAG-3' Cet oligonucléotide (20-mer) est en position 694 par rapport à l'adénosine 1 de l'ATG (initiation de la traduction du messager Epo). Cet oligonucléotide reconnaît la séquence inverse complémentaire de l'ARNm de l'Epo et borne la partie 3' de l'ADNc. . INTS 9 : 5'-AGGCGGGAGATGGGGTGC-3' Cet oligonucléotide (20-mer) est dans le même sens que l'ARNm et borne la partie 5' de l'ADNc de l'Epo (position-10 par rapport à l'adénosine 1 du codon d'initiation). La sonde INTS 10 permet de recopier la matrice ARNm de l'Epo en utilisant la reverse transcriptase. L'ARNm est ensuite dégradé à la soude pendant 1 heure à 65°C. Cet ADNc simple brin peut-être alors amplifié par la technique PCR en utilisant les deux sondes bornant l'ADNc : INTS 10 et INTS 9. Un fragment d'ADN de 723 pb a été ainsi amplifié puis isolé par électrophorèse, cloné dans un vecteur d'amplification du type pUC puis séquencé.

### 2.3. Introduction des séquences codantes de l'Epo dans le vecteur pTS39

La séquence codante de l'Epo (génomique ou ADNc) est introduite dans l'oligonucléotide (L₂) du plasmide pTS39 sous forme de fragment BstEII-BglII entre les sites HindIII et BglII.

Les constructions résultantes seront appelées pTS39-Epo.

### 2.4. Transfection des cellules et sélection des transformants

La transfection est réalisée avec un électropulsateur ATEIM (Bioblock, France). Deux solutions acqueuses sont préparées préalablement :
. Le milieu de fusion : Inositol 250 mM, KH₂PO₄ 1 mM, Mg Acétate 0,5 mM, Ca Acétate 0,1 mM ; pH 7,4.
. Le milieu post-fusion : NaCl 132 mM, KCI 8 mM, KH₂PO₄ 1mM, Mg Acétate 0,1 mM, Ca Acétate 0,1 mM ; pH 7,4.
Après 2 jours de culture, les cellules sont lavées et ajustées à 4.10⁷/ml dans le milieu de fusion. La suspension est incubée 10 min à 0°C en présence de l'ADN à transfecter (5µg/10⁶ cellules). Un échantillon de la suspension est placé entre les électrodes de l'appareil et l'électroporation est conduite dans les conditions suivantes: 4 impulsions rectangulaires à 1 seconde d'intervalle de durée 100 µs, avec un champ électrique de 1.500 V/cm². Immédiatement après le choc électrique la suspension est diluée au 1/20 puis incubée 20 min à 37°C. La viabilité des cellules se situe entre 20 et 60 %. Les cellules sont ensuite mises en culture à une concentration de 0,5 10⁶ cellules/ml.

48 heures après la transfection un aliquot du surnageant est prélevé pour le dosage de l'Epo. Les cellules sont alors cultivées en milieu sélectif (Iscove, 10 % SVF - sérum de veau foetal - dialysé, acide mycophénolique 1 µg/ml, xanthine 250 µg/ml correspondant au gène de sélection XGPRT).

Après 14 à 21 jours de sélection sur le pool, des clonages par dilution limite sont réalisés en microplaques de 96 puits en présence de cellules nourricières (lymphocytes humains totaux, irradiés à 4000 rds, issus de donneurs sains).

### 2.5. Production d'Epo HR par les cellules transformées

### ∗Cellules ATCC CCL156 transformés par le vecteur d'expression pTS39-Epo.

Environ 6.10⁶ cellules CCL156 sont transfectées par électroporation avec 30 µg d'ADN plasmidique puis soumises 48 h après transfection, au milieu sélectif contenant la xanthine et l'acide mycophénolique. La population résistante est clonée par dilution limite en présence de cellules nourricières (lymphocytes humains irradiés). Parmi les différents clones obtenus, le clone 156 0,5AF8 secrète environ 100 à 150 unités d'Epo par ml de milieu de culture/72 h et a été suivi à long terme en présence et en absence de pression de sélection. Les résultats indiquent que ce clone est stable sur 48 passages soit environ 7 mois de culture en absence de pression de sélection.

L'analyse en Southern blot de l'ADN total extrait du clone 156 0.5AF8 indique que le vecteur pTS39-Epo est intégré en tandem dans le génome cellulaire à raison de 15 à 20 copies par cellule, que le clone soit cultivé en présence ou en l'absence de pression de sélection.

En culture en "roller", le clone 0.5AF8 secrète 80 à 100 U Epo/ml en trois jours de culture en milieu pauvre en protéines CK4 ou CK4N (milieu de base : mélange Iscove-Ham F12 (1:1) ; CK4 : suppléments : albumine humaine 50 mg/l, transferrine humaine (saturée à 30 % par adjonction de FeC13) 1 mg/l, insuline bovine 3 mg/l, acide linoléique 1 mg/l, putrescine 10 µM, éthanolamine 20 µM ; CK4N, idem plus ribonucléotides : Adénosine 100 mg/l, Cytidine 100 mg/l, Guanosine 100 mg/l, Uridine 100 mg/l). Ce taux de sécrétion peut être renouvelé 3 fois si le milieu est changé tous les 3 jours.

### ∗Cellules immortalisées in vitro par l'EBV (lignée INTS-F5).

L'expérience réalisée sur les cellules CCL156 a été reproduite sur des cellules F5, déjà utilisées au laboratoire pour la production d'anticorps monoclonaux (Goosens et al. J. Immunol. Methods 1987, 101, 193) et qui sont transfectées avec le vecteur pTS39-Epo. Les dosages d'Epo effectués à différents temps après la transfection sont positifs : on détecte 29 U Epo/ml/10⁶ cellules après 21 jours de sélection.

### 2.6. Détection de l'activité biologique et immunologique de l'Epo HR

### ∗Culture d'érythroblastes de foie foetal.

L'activité de l'Epo est mesurée in vitro d'après l'incorporation de ⁵⁹Fe par les érythroblastes de foie foetal de souris (prélevés au 13è jour de gestation) cultivés en 26 heures selon une méthode dérivée de celle décrite par Stephenson et al, (Endocrinology 88, 1519, 1971).

Les cellules sont dissociées mécaniquement et mises en suspension en milieu RPMI 1640 (Tambourin et al, Biomedicine 19, 112, 1983 ; Goldwasser et al, Endocrinology 97, 315, 1975) à la concentration 1,6 x 10⁶ cellules/ml contenant 7 % de sérum de veau foetal, 85 µM d'albumine et 0,4 µM de transferrine humaine.

Selon les cas un milieu sans sérum est utilisé dans lequel des phospholipides naturels (soja, ovolecithine) purifiés ou synthétiques (32 x 10⁻³M) et du cholestérol (1,6 x 10⁻³M) sont solubilisés en chloroforme, secondairement évaporé sous courant d'azote. Les lipides en solution d'albumine à 1 mg/ml en RPMI sont dispersés par les ultrasons en glace fondante (Boffa et coll, Exp. Hématol. 10, 675, 1982).

La suspension cellulaire est répartie en volume de 200 µl dans les puits à fond rond des plaques de culture "Nunclon"^{M}. Après une incubation de 21 heures à 37°C en présence de 0,7% de CO₂, la ⁵⁹Fe-transferrine est incorporée dans les puits, les plaques sont agitées et remises en incubateur pour une incubation prolongée de 5 heures.

Chaque échantillon est analysé à 3 ou 4 concentrations protéiques. L'activité de chaque dose résulte d'une quadruple détermination. Le taux d'incorporation du ⁵⁹Fe sur l'hème extrait par la méthyléthylcetone ou dans les cellules est mesuré en fonction du logarithme de la dose de l'échantillon et les résultats sont exprimés en milliunité/ml ou par mg de protéine.

L'étalon est de l'Epo humaine de recombinaison titrant 100.000 U/mg. Son activité spécifique a été définie d'après les échantillons d'Epo humaine issus de la seconde préparation de Référence Internationale (Annable et al, Bull. Wld. Hlth. Org. 47, 99, 1972).

### ∗Culture de progéniteurs érythroïdes de moelle osseuse ou de sang périphérique.

Les effets de l'Epo HR sur la maturation des BFUe et des CFUe ont été déterminés en culture de précurseurs cellulaires érythroïdes sur méthylcellulose en milieu IMDM dépourvu de sérum (Cormier et al, Cell Différentiation, 17, 261, 1985). L'Epo HR, comme l'Epo humaine urinaire, détermine la différentiation et la maturation des BFUe issus de la moelle osseuse de souris ou du sang périphérique humain. L'effet-dose de l'Epo HR sur le développement des CFUe est comparé avec celui donné par l'Epo naturelle.

### ∗Détection par tests radio-immunologiques.

Le dosage radioimmunologique de l'Epo HR a été réalisé dans les surnageants cellulaires selon deux procédés:
. par précipitation par double anticorps en phase soluble : un immunsérum de lapin anti-Epo urinaire humaine monospécifique (Terry Fox Lab, Vancouver) est incubé avec un étalon d'Epo H ou un échantillon à doser pendant 16 h à 4°C. Après ce délai, 2000 cpm ¹²⁵I-Epo HR (Amersham) sont ajoutés et les préparations sont incubées 2 h à 4°C. La dilution de l'immunsérum choisie est celle qui donne une radioactivité fixée de 20 à 30 %. La dilution finale de l'immunsérum se situe entre 10⁻⁴ et 10⁻⁵. Après une incubation de 2 h à 20°C avec des immunoglobulines de chèvre anti-Fc de lapin, la radioactivité du culot est mesurée.
. par précipitation par double anticorps en phase solide sur Tachisorb^{M} :
   les réactifs sont répartis en un seul temps sur plaques de microtitration Dynatech^{M} 96 puits sous un volume final de 220µl. Après une incubation de 2 heures sous agitation horizontale rotative à la température ambiante, la récolte s'effectue sur filtre de fibres de verre sur appareil Skatron^{M}.

La distribution concerne successivement l'Epo de la gamme ou de l'échantillon sous un volume de 100 µl, l'immun sérum anti Epo 10 µl, 125-I-Epo HR 10 µl et le Tachisorb R 100 µl. Après transfert en tube de polystyrène des échantillons sur fibres de verre, la radioactivité est mesurée.

La gamme de mesure de la méthode est comprise entre 1 et 100mU.

Les surnageants des cellules transformées possèdent des activités pouvant atteindre 800 U/ml alors que les surnageants cellulaires avec vecteur anti-sens sont dépourvus d'activité. Il y a toujours une excellente correlation entre les dosages biologiques in vitro et le dosage radioimmunologique.

### 2.7. Détection et mesure de l'activité biologique in vivo de l'Epo HR

L'activité in vivo de l'Epo produite dans les surnageants de culture des cellules lymphoblastoïdes transformées est testée après injection à des souris rendues polyglobuliques par transfusion afin d'hinhiber la synthèse d'Epo endogène (Jacobson. et al Proc. Soc. Exp. Biol. Med, 94, 243, 1957). Les résultats indiquent que l'Epo produite par les cellules lymphoblastoïdes présente une puissante activité biologique comparable à celle de l'Epo HR produite par des cellules CHO (Brevet Kirin-Amgen EP 0 148 605).

Les résultats traduisent une étroite corrélation avec les dosages biologiques in vitro d'après la seconde préparation de référence internationale d'Epo urinaire humaine.

### Exemple 3 : Procédés de préparation de concentrés d'érythropoïétine purifiés à partir du surnageant des cultures de cellules lymphoblastoïdes transformées

### 3.a) Première méthode

Le surnageant de culture cellulaire en milieu pauvre en protéines (CK4) est concentré par ultrafiltration sur membrane PM10 Amicon ou par filtration tangentielle sur Minitan^{M} équipé d'une membrane PLGC suivie par un lavage en solution en glucose 12,5 mM, galactose 12,5 mM, PEG 6000 0,1 mg/ml β-mercaptoethanol 10⁻⁴ M, jusqu'à une résistivité du filtrat supérieure à 5000 Ohms.

Le concentrat lyophilisé est chromatographié sur Ultrogel^{M} AcA44 en tampon acétate de calcium 10 mM, NaCl 100 mM, phénol 5,6 10⁻⁴ M, glucose 12,5 mM, galactose 12,5 mM, PEG 6000 0,1 mg/ml pH 6,8. Les fractions actives sont concentrées par ultrafiltration et lyophilisées. Le produit est ensuite traité par HPLC sur colonne TSK 545 DEAE en tampon acétate 0,048 mM, CaCl₂ 3mM, glucose 12,5 mM, galactose 12,5 mM, PEG-6000 0,1 mg/ml, β-mercaptoethanol 10⁻⁴ M puis élution par un gradient de molarité discontinue de NaCl. Le rendement en activité est voisin de 75- %. Après dialyse et concentration, le produit est chromatographié sur CM Affigel blue^{M} en tampon phosphate 10mM, NaCl 150 mM pH 7,2. L'Epo est éluée par une molarité de 1,15 M NaCl. L'activité spécifique de l'Epo HR est supérieure à 100.000 U/mg.

### 3.b) Deuxième méthode

Le concentrat des milieux de culture obtenu sur membranes d'ultrafiltration est traité sur DEAE Séphacel^{M} à 4°C en tampon acétate 48 mM, CaCl₂ 3mM, phénol 5,4 10⁻⁴ M, pH 4,5. L'activité retenue est éluée en NaCl 0,6 M.

Après concentration et dialyse, l'éluat est soumis à une filtration sur Ultrogel^{M} AcA44, puis la fraction active détectée par RIA est chromatographiée en HPLC-DEAE en présence de réducteurs et d'hexoses stabilisants. L'Epo HR éluée en tampon pH 5,5, à faible molarité de NaCl, est concentrée sur membrane Amicon^{M} PM10 puis chargée sur une colonne de WGA-Sépharose 6MB en tampon PBS à 4°C. Son élution est obtenue par N-acetylglucosamine 0,5 M.
En électrophorèse sur gel en gradient de polyacrylamide (4 à 30 %) en présence de SDS, un composant de 34 kDa est observé dans les préparations issues du WGA-Sépharose. Ce composant est identifié en "immunoblot" par des immunoglobulines-polyclonales anti-Epo HR et des anticorps monoclonaux anti-Epo HR. Un composant minoritaire migrant sur une zone de 42 kDa est observé dans certaines préparations. Ce composant n'est pas reconnu en immunoblots par des immuns sérums polyclonaux anti-Epo HR. Ce résultat est confirmé par la détection radioimmunologique et par l'analyse en culture d'érythroblastes de foie foetal de souris des éluats d'électrophorèse préparative en gel de polyacrylamide SDS.
Selon les cas, une étape de purification complémentaire est réalisée soit sur CM Affigel blue^{M}, soit sur HA-Ultrogel^{M} ou en HPLC filtration en milieu SDS 0,1 %. Le poids moléculaire apparent de l'Epo HR en gel de polyacrylamide/SDS est de 34 kDa.

L'activité spécifique obtenue est supérieure à 120.000 U/mg en culture d'érythroblastes et en RIA avec des rendements compris entre 20 et 30 %.

### 3.c) Troisième méthode : Immunopurification de l'Epo HR

### ∗c-1. Préparation de l'immunoadsorbant

A partir de surnageants cellulaires la préparation d'Epo HR purifiée a été réalisée d'après la méthode décrite dans le deuxième exemple ci-dessus. L'activité spécifique de cette préparation déterminée par dosage radioimmunologique et par son activité en culture d'érythroblastes était de 130.000 U.I.

l'Epo HR lyophilisée puis reprise en solution en présence de SDS à 0,05 % a servi à l'immunisation de souris Balb/c.

Chaque souris a reçu à 15 jours d'intervalle trois injections intrapéritonéales aux doses suivantes : 90, 45 et 10 ou 35 µg pour la dernière injection. Toutes les fusions ont été réalisées 5 jours après la dernière injection.

Selon les expériences 30 à 90 % des puits de fusion ont été testés. Les anticorps sont recherchés dans les surnageants des puits de fusion d'après leur capacité de fixer 1'¹²⁵I-Epo HR. L'identification des complexes marqués est obtenue par un anticorps de chèvre anti-IgG de souris fixé par liaison covalente sur protéine A de staphylocoques. Les complexes sont collectés et comptés.

Au cours de la 10ème fusion, 3 hybrides se sont révélés positifs et ont été clonés. Le sérum de la souris qui a fourni les splénocytes présentait une réactivité immunologique de 65 % avec une dilution au 1/3000. Un anticorps monoclonal anti-Epo de référence : Ep10-141A26 de classe IgG a été obtenu. Son taux de fixation est compris entre 50 et 60 %. Le clone producteur de l'anticorps s'est avéré stable après de multiples clonages et sous clonages.

Une expansion réalisée en culture et en ascite a permis d'obtenir des volumes suffisants pour la purification de plusieurs dizaines de milligrammes d'anticorps. La purification des anticorps est réalisée sur protéine G-Sépharose. L'anticorps purifié est obtenu par adsorption de l'ascite en tampon phosphate 0,1 M, pH 7,5, lavage à pH 6,5 et élution en tampon glycine-HCl 0,1 M, pH 2,8. Des lots de 30 mg d'anticorps Ep10-141A26 purifié sont obtenus et fixés sur Sépharose 4B activité (8ml de gel) par le bromure de cyanogène. A titre d'exemple une telle colonne peut fixer environ 5 à 6 mg d'Epo HR. Des essais préliminaires ont montré que l'Epo était adsorbée par l'anticorps immobilisé à un pH voisin de la neutralité. Son élution a été analysée à différents pH : 6,5, 4,5, 2,8 et 2,2 ; elle s'effectue principalement à partir de pH 4,5 jusqu'à 2,8.

La conservation de l'immunoadsorbant est effectuée en tampon phosphate pH 7,5, Thymirosal^{M} 0,02 % à 4°C ; la colonne peut être utilisée pour de nombreux cycles.

### ∗c-2. Purification de l'Epo

La purification est réalisée en deux étapes :
- Première étape :
   Le surnageant de cellules lymphoblastoïdes est amené par ultrafiltration à une concentration protéique de 3 à 4 mg/ml, puis dialysé en eau déminéralisée et équilibré avec l'acide acétique à pH 4,5 et à 1.000 Ohms par addition d'eau.
   Après élimination d'un précipité par centrifugation le produit est traité sur colonne de DEAE Séphacel^{M}, équilibré en tampon acétate 0,04 M, CaCl₂ 0,0025 M et phénol 5,4 10⁻⁴ M, avec un rapport égal à 0,1 (volume d'échangeur en ml/protéines incorporées en mg).
   L'élution de la fraction active obtenue par addition de 0,6 M NaCl dans le tampon de départ représente 15 à 20 % des protéines incorporées. Les produits élués sont équilibrés à pH 7 puis dialysés jusqu'à une résistivité de 80 à 100 Ohms.
- Deuxième étape :
   Elle est réalisée sur l'immunoadsorbant préparé selon la méthode décrite ci-dessus. Les protéines éluées de la DEAE Séphacel^{M} sont incorporées sur l'immunoadsorbant en tampon phosphate 0,1 M pH 7,5 à 4°C avec un débit de 6 à 7 ml/heure. Le lavage est effectué à pH 6,5 dans le même tampon. L'élution de l'Epo a été obtenue par un tampon glycine 0,1 M HCl pH 2,2 avec un débit de 50 ml/heure à la température ambiante. L'éluat est équilibré par du tampon tris 1 M, pH 7,5. Le rendement en activité se situe entre 50 et 60 % de l'Epo active de départ.

### ∗c-3. Caractéristiques de l'Epo HR immunopurifiée à partir des produits des cellules lymphoblastoïdes.

Le degré d'homogénéité des préparations d'Epo HR est apprécié par densitométrie après électrophorèse en gel de polyacrylamide SDS. L'Epo est homogène à plus de 98 %. Le poids moléculaire apparent mesuré par électrophorèse en gel de polyacrylamide SDS est de 34.000 daltons. Son comportement électrophorétique est identique à celui de l'Epo produite par des cellules CHO ainsi qu'à celui de la molécule radioiodée (commercialisée par Amersham). Son point isoélectrique est compris entre 3,5 et 4. Son activité spécifique est supérieure aux préparations d'Epo urinaire et se situe selon les lots aux environs de 200.000 UI/mg.

### Exemple 4 : Utilisation du plasmide vecteur intégratif pour produire l'Interleukine-3 humaine dans les cellules lymphoblastoïdes.

Une sonde constituée de l'oligonucléotide complémentaire inverse (30-mer) d'une séquence de l'IL-3 : 5'TAA GTG TGT TAT AAT TTC ATC GAT CAT GTT 3' a été utilisée pour cribler une banque d'ADN génomique de leucocytes humains et une banque d'ADNc de foie foetal humain. La sonde synthétisée correspond à la base 110 (et suivantes) en aval de l'ATG initiateur de la séquence codante ADNc de l'IL-3.

Plusieurs clones ont été isolés et la présence du gène a été confirmée par cartographie de restriction. Les phages contenant ces gènes sont sous-clonés dans pUC 18 sous forme de fragment Acc I de 5,4 Kb. Avant d'introduire ce fragment dans la cassette d'expression du plasmide vecteur, les séquences promoteur et 3' non traduite en sont éliminées. Pour cela, la région contenant la séquence codante est amplifiée par la méthode PCR à l'aide des 2 oligonucléotides de synthèse suivants : INTS 16 : 5' GATCCAAACATGAGCCGCCT 3' début : position -9 de l'ATG (Met initiatrice) INTS 17 : 5' ATGTCCCGAGGCGCTGTGGT 3' début : position 52 en aval du codon stop
en utilisant comme matrice les clones génomiques dans pUC18, caractérisés précédemment.

Le produit d'amplification de 2.140 pb est sous-cloné dans le site Sma I d'un pUC18 et partiellement séquencé pour vérifier l'intégrité du gène IL-3 (selon Yang et Clark, dans "Developmental Control of globin gene expression" A.R. Liss p.3-11 1987).

La séquence codante ainsi contrôlée a été introduite dans la cassette d'expression du plasmide vecteur décrit dans l'exemple 1. Un clone cellulaire transformé donnant une réponse bien positive a été sélectionné et multiplié.

De l'interleukine-3 biologiquement active est sécrétée dans le milieu de culture des cellules.

### Exemple 5 : Utilisation du plasmide vecteur intéaratif pour produire du GM-CSF humain dans les cellules lymphoblastoïdes.

Une sonde constituée de l'oligonucléotide complémentaire inverse (30 mer) d'une séquence du GM-CSF : 5' TTG CTC TAA GCA CTT GAG CAG GAA GCT CTG 3' a été utilisée pour cribler une banque d'ADN génomique de leucocytes humains et une banque d'ADNc de foie foetal humain. La sonde synthétisée correspond à la base 157 (et suivantes) en aval de l'ATG initiateur de la séquence codante du GM-CSF.

Plusieurs clones ont été isolés et la présence du gène a été confirmée par cartographie de restriction. Les phages contenant ces gènes sont sous-clonés dans pUC18 sous forme de fragment Hind III/Eco RI de 3,2 Kb. Avant d'introduire ce fragment dans la cassette d'expression du plasmide vecteur, les séquences promoteur et 3' non traduite en sont éliminées. Pour cela, la région contenant la séquence codante est amplifiée par la méthode PCR à l'aide des 2 oligonucléotides de synthèse suivants : INTS 19 : 5'CACAGAGAGAAAGGCTAAAG 3' début : position-31 de l'ATG (Met initiatrice) INTS 18 : 5' ATTCCCATTCTTCTGCCATG 3' début : position 177 en aval du codon stop
en utilisant comme matrice les clones génomiques dans pUC18, caractérisés précédemment.

Le produit d'amplification de 2.240 pb est sous-cloné dans le site Sma I d'un pUC18 et partiellement séquencé pour vérifier l'intégrité du gène GM-CSF (selon Miyatake et al. EMBO J.4, 2561, 1985).

La séquence codante ainsi contrôlée a été introduite dans la cassette d'expression du plasmide vecteur décrit dans l'exemple 1. Un clone cellulaire transformé donnant une réponse bien positive a été sélectionné et multiplié.

Du GM-CSF biologiquement actif est sécrété dans le milieu de cultures des cellules.

## Revendications

1. Utilisation de lignées de cellules lymphoblastoïdes humaines, immortalisées in vitro par le virus d'Epstein-Barr (EBV) et sélectionnées pour leur absence de caractère tumoral ou tumorigène, pour la production à l'échelle industrielle de protéines hétérologues codées par un segment d'ADN inséré dans un plasmide vecteur génétiquement manipulé, de type intégratif et portant une cassette d'expression comprenant tous les éléments permettant l'expression desdites protéines hétérologues dans lesdites cellules lymphoblastoïdes après intégration de ladite cassette d'expression dans l'ADN chromosomique cellulaire.

2. Plasmide vecteur destiné à l'utilisation des cellules lymphoblastoïdes pour la production de protéines hétérologues selon la revendication 1, comportant les éléments d'ADN bactérien nécessaires à son amplification dans un hôte bactérien de type E.coli, caractérisé en ce qu'il comporte simultanément :
a) les éléments d'ADN constituant une cassette d'expression d'un ADN hétérologue, ladite cassette étant conçue en vue de son utilisation dans les cellules lymphoblastoïdes humaines,
b) la séquence d'ADN de l'Adenovirus 5 codant pour les ARN VAI et VAII, pour optimaliser la traduction des ARN messagers,
c) un gêne de sélection manipulé pour être efficace dans les cellules eucaryotes,
d) des éléments d'ADN sélectionnés pour favoriser l'intégration de l'ADN plasmidique dans l'ADN chromosomique cellulaire.

3. Plasmide vecteur selon la revendication 2 caractérisé en ce que les éléments d'ADN constituant une cassette d'expression incluse dans ledit plasmide vecteur comprennent :
a) une séquence activatrice de transcription dérivée d'un Adenovirus,
b) un promoteur et une séquence "leader" dérivés d'un Adenovirus,
c) un oligonucléotide de synthèse contenant des sites de restriction destinés à l'insertion de la séquence d'ADN codant pour une protéine hétérologue choisie,
d) un signal de polyadénylation dérivé du virus SV 40.

4. Plasmide vecteur selon la revendication 2 ou 3 caractérisé en ce que, dans la cassette d'expression :
- l'élément a) est la séquence activatrice du gène EIA de l'Adenovirus 5 humain ;
- l'élément b) est le promoteur majeur tardif de l'Adenovirus 2 humain associé à la séquence "leader" tripartite du même Adenovirus 2 ;
- l'élément c) a été conçu pour offrir des sites de restriction rares pour favoriser la manipulation d'insertion de la séquence d'ADN hétérologue.

5. Plasmide vecteur selon l'une quelconque des revendications 2 à 4 caractérisé en ce que l'oligonucléotide inclus dans la cassette d'expression offre les sites de restriction Not I, Bst EII, Bgl II, MLu I.

6. Plasmide vecteur selon l'une quelconque des revendications 2 à 5 caractérisé en ce que le gène de sélection est choisi parmi - l'ADN codant pour la xanthine-guanine phosphoribosyl transférase (XGPRT) de E-coli placé sour le contrôle du promoteur et des signaux de maturation du gène Tk du virus HSV1,
- l'ADNc de la dihydrofolate réductase (DHFR) de souris
- ou la combinaison des deux, insérés à des points distincts du plasmide vecteur.

7. Plasmide vecteur selon l'une quelconque des revendications 2 à 6 caractérisé en ce que les éléments d'ADN inclus dans ledit plasmide pour en favoriser l'intégration comprennent un fragment d'ADN mitochondrial de souris.

8. Plasmide vecteur selon l'une quelconque des revendications 2 à 7 caractérisé en ce que l'ADN hétérologue inclus dans la cassette d'expression est constitué de la séquence codante d'une protéine choisie parmi les protéines d'intérêt thérapeutique difficiles à purifier à partir d'une source naturelle.

9. Plasmide vecteur selon l'une quelconque des revendications 2 à 8 caractérisé en ce que l'ADN hétérologue inclus dans la cassette d'expression est constitué de la séquence codante de l'érythropoïétine ou d'une protéine ayant l'activité de l'érythropoïétine.

10. Plasmide vecteur selon l'une quelconque des revendications 2 à 8 caractérisé en ce que l'ADN hétérologue inclus dans la cassette d'expression est constitué de la séquence codante de l'interleukine 3 ou d'une protéine ayant l'activité de l'interleukine 3.

11. Plasmide vecteur selon l'une quelconque des revendications 2 à 8 caractérisé en ce que l'ADN hétérologue inclus dans la cassette d'expression est constitué de la séquence codante du GM-CSF.

12. Lignées cellulaires lymphoblastoïdes humaines immortalisées par l'EBV pour l'utilisation selon la revendication 1, caractérisées en ce qu'elles sont transformées de manière stable par les éléments d'ADN d'un plasmide vecteur selon l'une quelconque des revendications 2 à 12.

13. Lignées cellulaires selon la revendication 12, caractérisées en ce qu'elles sont clonées et sélectionnées par criblage pour leur taux élevé de sécrétion de la protéine hétérologue choisie.

14. Lignées cellulaires selon la revendication 12 ou 13, caractérisées en ce que la lignée de départ réceptrice du plasmide vecteur est la lignée déposée à l'ATCC sous la référence CCL 156 RPMI 1788.

15. Lignées cellulaires selon la revendication 12 ou 13, caractérisées en ce que la lignée de départ réceptrice du plasmide vecteur est une lignée dérivée de lymphocytes B d'un donneur sain, par immortalisation par l'EBV.

## Patentansprüche

1. Verwendung von Human-Lymphoblastoid-Zellinien, die mit dem Epstein-Barr-Virus (EBV) in vitro immortalisiert und aufgrund des Fehlens eines tumoralen oder Tumor-bildenden Charakters selektioniert worden sind, für die Herstellung von heterologen Proteinen in industriellem Maßstab, die durch ein DNA-Segment codiert sind, das in ein genetisch manipuliertes Vektor-Plasmid vom integrativen Typ inseriert worden ist, das eine Expressions-Kassette trägt, die alle Elemente enthält, welche die Expression der genannten heterologen Proteine in den genannten Lymphoblastoid-Zellen nach der Integration der genannten Expressions-Kassette in die zelluläre Chromosomen-DNA erlaubt.

2. Vektor-Plasmid für die Verwendung von Lymphoblastoid-Zellen zur Herstellung von heterologen Proteinen nach Anspruch 1, das die für seine Amplifikation in einem Wirts-Bakterium vom Typ E. coli erforderlichen Elemente einer Bakterien-DNA enthält, dadurch gekennzeichnet, daß es gleichzeitig enthält:
a) die DNA-Elemente, die eine Expressions-Kassette einer heterologen DNA aufbauen, wobei die genannte Kassette konzipiert ist im Hinblick auf ihre Verwendung in den Human-Lymphoblastoid-Zellen,
b) die DNA-Sequenz des Adenovirus 5, die für die RNA VAI und VAII codiert, um die Translation der Messenger-RNA zu optimieren,
c) ein Selektions-Gen, das so manipuliert ist, daß es in den Eukaryontenzellen wirksam ist, und
d) DNA-Elemente, die so selektioniert worden sind, daß sie die Integration der Plasmid-DNA in die zelluläre Chromosomen-DNA begünstigen.

3. Vektor-Plasmid nach Anspruch 2, dadurch gekennzeichnet, daß die DNA-Elemente, die eine in dem genannten Vektor-Plasmid enthaltene Expressions-Kassette aufbauen, umfassen:
a) eine Transkriptions-Aktivierungs-Sequenz, die von einem Adenovirus abgeleitet ist,
b) einen Promotor und eine "Leader"-Sequenz, die von einem Adenovirus abgeleitet sind,
c) ein Synthese-Oligonucleotid, das Restriktionsstellen enthält, die für die Insertion der DNA-Sequenz, die für ein ausgewähltes heterologes Protein codiert, bestimmt sind, und
d) ein Polyadenylierungs-Signal, das von dem Virus SV 40 abgeleitet ist.

4. Vektor-Plasmid nach Anspruch 2 oder 3, dadurch gekennzeichnet, daß in der Expressions-Kassette
- das Element a) die Aktivierungs-Sequenz für das Gen EIA des Human-Adenovirus 5 ist;
- das Element b) der "späte" Haupt-Promotor des Human-Adenovirus 2 ist, der mit der dreigeteilten "Leader"-Sequenz des gleichen Adenovirus 2 assoziiert ist;
- das Element c) so konzipiert ist, daß es wenige Restriktionsstellen anbietet, um die Manipulation der Insertion der heterologen DNA-Sequenz zu begünstigen.

5. Vektor-Plasmid nach einem der Ansprüche 2 bis 4, dadurch gekennzeichnet, daß das in der Expressions-Kassette enthaltene Oligonucleotid Restriktionsstellen Not 1, Bst Ell, Bgl II, MLu I anbietet.

6. Vektor-Plasmid nach einem der Ansprüche 2 bis 5, dadurch gekennzeichnet, daß das Selektions-Gen ausgewählt wird aus der Gruppe
- DNA, die für die Xanthinguaninphosphoribosyl-Transferase (XGPRT) von E. coli codiert, die unter der Kontrolle des Promotors und der Reifungssignale des Tk-Gens des Virus HSV1 steht,
- cDNA der Dihydrofolat-Reduktase (DHFR) der Maus oder
- der Kombination der beiden, die an verschiedenen Punkten des Vektor-Plasmids inseriert sind.

7. Vektor-Plasmid nach einem der Ansprüche 2 bis 6, dadurch gekennzeichnet, daß die DNA-Elemente, die in dem genannten Plasmid enthalten sind, um dessen Integration zu begünstigen, ein Mitochondrien-DNA-Fragment der Maus enthalten.

8. Vektor-Plasmid nach einem der Ansprüche 2 bis 7, dadurch gekennzeichnet, daß die in der Expressions-Kassette enthaltene heterologe DNA besteht aus der codierenden Sequenz eines Proteins, das ausgewählt wird unter den Proteinen von therapeutischem Interesse, die, ausgehend von einer natürlichen Quelle, schwierig zu reinigen sind.

9. Vektor-Plasmid nach einem der Ansprüche 2 bis 8, dadurch gekennzeichnet, daß die in der Expressions-Kassette enthaltene heterologe DNA besteht aus der codierenden Sequenz von Erythropoietin oder einem Protein mit einer Erythropoietin-Aktivität.

10. Vektor-Plasmid nach einem der Ansprüche 2 bis 8, dadurch gekennzeichnet, daß die in der Expressions-Kassette enthaltene heterologe DNA besteht aus der codierenden Sequenz von Interleukin 3 oder einem Protein, das die Aktivität von Interleukin 3 hat.

11. Vektor-Plasmid nach einem der Ansprüche 2 bis 8, dadurch gekennzeichnet, daß die in der Expressions-Kassette enthaltene heterologe DNA aus der codierenden Sequenz von GM-CSF besteht.

12. Durch EBV immortalisierte Human-Lymphoblastoid-Zellinien für die Verwendung nach Anspruch 1, dadurch gekennzeichnet, daß sie durch die DNA-Elemente eines Vektor-Plasmids nach einem der Ansprüche 2 bis 12 auf stabile Weise transformiert worden sind.

13. Zellinien nach Anspruch 12, dadurch gekennzeichnet, daß sie kloniert und selektioniert worden sind durch Aussortieren aufgrund ihrer erhöhten Sekretion des ausgewählten heterologen Proteins.

14. Zellinien nach Anspruch 12 oder 13, dadurch gekennzeichnet, daß die Ausgangs-Zellinie, die das Vektor-Plasmid aufnimmt, die bei ATCC unter der Referenz-Nr. CCL 156 RPMI 1788 hinterlegte Zellinie ist.

15. Zellinien nach Anspruch 12 oder 13, dadurch gekennzeichnet, daß die Ausgangs-Zellinie, die das Vektor-Plasmid aufnimmt, eine Zellinie ist, die von den Lymphozyten B eines gesunden Spenders durch Immortalisierung mit EBV gewonnen worden ist.

## Claims

1. The use of human lymphoblastoid cell lines which have been immortalized in vitro by Epstein-Barr virus (EBV) and selected for their absence of tumoral or tumorigenic character, for producing, on an industrial scale, heterologous proteins encoded by a DNA fragment inserted into a genetically engineered vector plasmid, of the integrative type, bearing an expression cassette comprising all the elements allowing the expression of said heterologous proteins in said lymphoblastoid cells once said expression cassette is integrated into the cell chromosome DNA.

2. The vector plasmid designed for using the lymphoblastoid cells according to claim 1 for producing heterologous proteins, which comprises the bacterial DNA elements required for its amplification characterized in that it simultaneously comprises the following DNA fragments :
- the DNA elements which constitute an expression cassette allowing the insertion of an heterologous DNA to be expressed in the human lymphoblastoid cells,
- the Adenovirus 5 derived DNA sequence encoding the VA I and VA II RNA aimed at optimizing the messenger RNA translation,
- a gene engineered to be efficient as a selective marker in eukaryotic cells,
- DNA segments selected for their property to promote plasmid DNA integration into the cell chromosme DNA.

3. The vector plasmid according to claim 2 characterized in that the DNA elements which form an expression cassette integrated into said vector comprise :
a) a transcription activator sequence derived from an Adenovirus,
b) a promotor and a leader sequence derived from an Adenovirus,
c) a synthetic oligonucleotide including restriction sites aimed at integrating the DNA coding sequence of the heterologous protein,
d) a polyadenylation signal derived from SV40 virus.

4. The vector plasmid according to claim 2 or 3 characterized in that the expression cassette comprises :
- as the element a), the activator sequence of the EIA gene of human Adenovirus 5,
- as the element b), the major late promotor of Adenovirus 2 coupled with the tripartite leader sequence of the same Adenovirus 2,
- as the element c), a sequence designed to provide rare restriction sites in order to facilitate the integration of the heterologous sequence.

5. The vector plasmid according to any of claims 2 to 4 characterized in that the oligonucleotide inserted into the expression cassette presents the restriction sites Not I, Bst EII, Bgl IT, MLuI.

6. The vector plasmid according to any of claims 2 to 5 characterized in that the gene engineered to be efficient as a selective marker is chosen among :
- the E.coli DNA coding for xanthine-guanine-phosphoryl-transferase (XGPRT) inserted under the control of Herpes simplex virus 1 Tk gene promotor and maturation signals,
- the mouse dihydrofolate reductase (DHFR) cDNA,
- or a combination of both genes, inserted at different locations in the vector plasmid.

7. The vector plasmid according to any of claims 2 to 6 charactarized in that the DNA segments selected for their property to promote said plasmid DNA integration comprise a fragment of mouse mitochondrial DNA.

8. The vector plasmid according to any of claims 2 to 7 characterized in that the heterologous DNA inserted in the expression cassette is chosen among the coding sequences of therapeutically useful proteins which are hard to purify from natural sources.

9. The vector plasmid according to any of claims 2 to 8 characterized in that the heterologous DNA inserted in the expression cassette is the coding sequence of erythropoietin or a protein having erythropoietin activity.

10. The vector plasmid according to any of claims 2 to 8 characterized in that the heterologous DNA inserted in the expression cassette is the coding sequence of interleukin-3 or a protein having interleukin-3 activity.

11. The vector plasmid according to any of claims 2 to 8 characterized in that the heterologous DNA inserted in the expression cassette is the coding sequence of GM-CSF.

12. Human lymphoblastoid cell lines immortalized by EBV, for the use according to claim 1, characterized in that they are stably transformed by the DNA elements of a vector plasmid according to any of claims 2 to 11.

13. The human lymphoblastoid cell lines according to claim 12 characterized in that they are cloned and screened for their high level of secretion of the chosen heterologous protein.

14. The human lymphoblastoid cell lines according to claims 12 or 13 characterized in that the starting cell line to be transformed by the vector plasmid is the ATCC-CCL 156 RPMI 1788 cell line.

15. The human lymphoblastoid cell lines according to claims 12 or 13 characterized in that the starting cell line to be transformed by the vector plasmid is a cell line derived from B lymphocytes of a healthy donor and immortalized by EBV.
